(19) Europäisches Patentamt
European Patent Office
Office européen des brevets

(11) **EP 2 711 696 B1**

(12) **FASCICULE DE BREVET EUROPEEN**

(45) Date de publication et mention
de la délivrance du brevet:
**09.06.2021 Bulletin 2021/23**

(51) Int Cl.:
*G01N 25/18* (2006.01)   *G01N 27/18* (2006.01)
*G01N 29/02* (2006.01)   *B81B 3/00* (2006.01)
*G01N 30/66* (2006.01)   *G01N 33/00* (2006.01)

(21) Numéro de dépôt: **13185043.0**

(22) Date de dépôt: **18.09.2013**

(54) **Capteur de flux thermique à élément vibrant et capteur de gaz comportant au moins un tel capteur**

Wärmeflusssensor mit Schwingelement und Gas-Sensor mit wenigstens einem solchen
Wärmeflusssensor

Thermal flow sensor with vibrating element and gas sensor with at least one such thermal flow sensor

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **19.09.2012 FR 1258795**

(43) Date de publication de la demande:
**26.03.2014 Bulletin 2014/13**

(73) Titulaire: **Commissariat à l'Énergie Atomique
et aux Énergies Alternatives
75015 Paris (FR)**

(72) Inventeurs:
• **Duraffourg, Laurent**
  **38500 Voiron (FR)**
• **Andreucci, Philippe**
  **38430 Moirans (FR)**
• **Colinet, Eric**
  **38000 Grenoble (FR)**
• **Jourdan, Guillaume**
  **38000 Grenoble (FR)**
• **Arcamone, Julien**
  **38000 Grenoble (FR)**

(74) Mandataire: **Ahner, Philippe
BREVALEX
95, rue d'Amsterdam
75378 Paris Cedex 8 (FR)**

(56) Documents cités:
**FR-A1- 2 965 349     US-A1- 2011 077 872**

• **XIAOBO GUO ET AL: "Gas sensing using
thermally actuated dual plate resonators and
self-sustained oscillators", FREQUENCY
CONTROL SYMPOSIUM (FCS), 2012 IEEE
INTERNATIONAL, IEEE, 21 mai 2012 (2012-05-21),
pages 1-5, XP032205290, DOI:
10.1109/FCS.2012.6243727 ISBN:
978-1-4577-1821-2**
• **D. MERCIER ET AL: "Characterization of a
SAW-Pirani vacuum sensor for two different
operating modes", SENSORS AND ACTUATORS
A: PHYSICAL, vol. 188, 1 février 2012
(2012-02-01), pages 41-47, XP055082098, ISSN:
0924-4247, DOI: 10.1016/j.sna.2012.01.039**
• **ARCAMONE J ET AL: "VLSI silicon multi-gas
analyzer coupling gas chromatography and
NEMS detectors", ELECTRON DEVICES
MEETING (IEDM), 2011 IEEE INTERNATIONAL,
IEEE, 29.3.1, 5 décembre 2011 (2011-12-05),
pages 669-672, XP032096029, DOI:
10.1109/IEDM.2011.6131637 ISBN:
978-1-4577-0506-9**
• **ASHISH KUMAR NAMDEO ET AL: "FEM Study on
Contactless Excitation of Acoustic Waves in SAW
Devices", PROCEEDINGS OF THE 2009 COMSOL
CONFERENCE, 14 November 2009 (2009-11-14),
pages 1-4, XP055311026,**

EP 2 711 696 B1

**Description**

**DOMAINE TECHNIQUE ET ART ANTÉRIEUR**

La présente invention se rapporte à un capteur de flux thermique mettant en œuvre au moins un résonateur nanoélectromécanique (ou NEMS pour "nanoelectromechanical system" en anglais) ou microélectromécanique (ou MEMS pour "microelectromechanical system" en anglais), pour déterminer la concentration des composants d'un gaz à partir de ses caractéristiques thermiques, et au capteur de gaz comportant au moins un tel capteur.

On entend par capteur de flux thermique, tout capteur mesurant un échange de chaleur entre le corps du capteur et le milieu fluidique dans lequel le capteur est disposé. Ce capteur de flux thermique est par exemple un capteur de gaz ou un capteur de pression.

Un système apte à déterminer la composition en analyte d'un gaz peut être utilisé pour détecter et quantifier les analytes à la sortie d'une colonne de chromatographie, plus particulièrement une microcolonne de chromatographie cette dernière permettant de séparer temporellement les différents éléments gazeux d'un mélange complexe. Le capteur sert à quantifier la concentration relative des analytes du gaz à analyser arrivant successivement à sa surface. Les analytes sont mélangés dans un gaz, dit gaz porteur, qui est envoyé dans la colonne de chromatographie et sur le capteur à une vitesse fixée.

Le gaz porteur peut être de l'air sec ou un gaz inerte par exemple.

Il existe plusieurs types de capteur pouvant être disposés en sortie de la colonne de chromatographie.

Les capteurs par ionisation à flamme ou FID (Flame ionization detector) en terminologie anglaise).

Les gaz à analyser sont brûlés sous flux d'hydrogène créant des ions et des électrons. Les particules chargées sont collectées par des électrodes et le courant généré est mesuré avec un pico-ampèremètre. D'une part, ce capteur ne permet que la détection de composants organiques. D'autre part, il requiert un flux d'hydrogène et la quantité produite d'ions restent faibles. Enfin la taille du capteur ne peut être réduite.

Il existe également des capteurs optiques dont le principe de fonctionnement est généralement basé sur l'absorption infrarouge d'un flux optique. Ces capteurs sont adaptés à la détection d'éléments carbonés. Mais pour pouvoir détecter d'autres types de gaz le nombre de sources laser devrait être multiplié, ce qui augmenterait fortement la complexité et le coût d'un tel appareil. Ces capteurs sont également difficilement miniaturisables.

Des capteurs électroniques dont le principe de détection est basé sur la variation d'une propriété électrique (résistance électrique, résistance, potentiel de surface) induite par la présence des molécules de gaz à sa surface. Ces capteurs nécessitent une fonctionnalisation de surface. Les capteurs macroscopiques sont relativement peu sensibles. Les capteurs de taille micrométrique ou nanométriques souffrent, quant à eux, de problème de drift, i.e. de dérives aléatoires long terme de la grandeur physique à mesurer et d'une extrême sensibilité aux états de surfaces initiaux. Ils doivent en outre être fonctionnalisés.

Il existe également des capteurs de conductivité thermique dit TCD pour "Thermal Conductivity Detectors". Un détecteur TCD peut comporter un fil porté à haute température dont on mesure la résistance électrique. Le fil a une température donnée pour un gaz donné. Lorsque le gaz change, les propriétés de l'environnement thermique (conductance thermique, viscosité, convection thermique) changent, ce qui provoque une variation de la température du fil. Cette variation induit elle-même un changement de résistance électrique qui est détectée à travers un pont de mesure. Plus la température du capteur TCD est élevée, meilleure est sa résolution. Il est donc nécessaire de travailler dans un environnement sans oxygène pour éviter que le fil ne brûle. Généralement il faut placer le fil TCD sous un flux de gaz porteur hélium ou sous un flux de gaz porteur hydrogène. L'utilisation d'un gaz rare permet d'avoir un fort contraste par rapport à l'air. Ceci représente une limitation importante du détecteur. En outre il existe un fort contraste des constantes thermiques entre ces gaz légers et les analytes à détecter ce qui rend le système plus sensible que sous un simple flux d'air sec.

Il existe également des capteurs gravimétriques. Il s'agit ici de mesurer la quantité de masse du gaz cible adsorbée à la surface du capteur.

Généralement le capteur est un système vibrant à une fréquence propre d'oscillation. On mesure le décalage en fréquence d'oscillation du système qui résulte de l'ajout de masse également appelé par effet gravimétrique, vers les basses fréquences provoqué par l'adsorption du gaz. La surface du capteur est fonctionnalisée pour capter le gaz. Ces capteurs présentent une sensibilité élevée pour les grosses molécules gazeuses mais une sensibilité moindre en mesure de concentration pour des molécules très légères et/ou volatiles. Ce type de capteur est décrit dans le document Fanget, S. Hentz, P. Puget, J. Arcamone, M. Matheron, E. Colinet, P. Andreucci, L. Duraffourg, E. Myers, M.L. Roukes, "Gas sensors based on gravimétrie détection - A review", Sensors and Actuators, B: Chemical, 160(1), 2011, 804-821

D. Mercier et al., "Characterization of a SAW-Pirani vacuum sensor for two différent operating modes", Sensors and actuators, A: Physical, 188 (2012) 41-47 décrit un capteur de flux thermique comportant un élément vibrant seulement à sa surface.

## EXPOSÉ DE L'INVENTION

C'est par conséquent un but de la présente invention d'offrir un capteur de flux thermique offrant une grande sensibilité et apte à être miniaturisé.

Le but de la présente invention est atteint par un capteur de flux thermique comportant un élément vibrant apte être mis en vibration par des moyens d'excitation, des moyens de chauffage dudit élément vibrant et des moyens de mesure de la variation de fréquence de l'élément vibrant de sorte à déterminer une variation de température et ainsi un flux thermique.

L'élément vibrant est disposé dans le gaz à analyser. L'élément vibrant est suspendu par rapport à un support de sorte à limiter les pertes thermiques de l'élément vibrant vers le support. Les moyens de suspension forment alors des moyens d'isolation thermique. De manière avantageuse, les moyens de suspension et d'isolation thermique sont formés par des poutres de type nanofil.

Dans un mode de réalisation, les moyens de chauffage sont en contact avec l'élément vibrant, ce contact étant électrique et mécanique

Dans un autre mode de réalisation, les moyens de chauffage sont situés à distance de l'élément vibrant, ce dernier étant échauffé par conduction thermique à travers le gaz à analyser.

En d'autres termes, on mesure la variation de la fréquence de vibration de l'élément vibrant qui est induit par la variation de température de l'élément vibrant du fait des échanges thermiques avec le gaz environnant. A partir de cette variation de fréquence de vibration, il est possible de déterminer les flux thermiques entre l'élément vibrant et le gaz et en déduire la composition du gaz qui est responsable de ces échanges thermiques, dans le cas d'un capteur de gaz.

Ce capteur présente l'avantage d'être insensible à l'absorption de masse puisque du fait de l'échauffement de l'élément vibrant, la masse absorbée est immédiatement désorbée et n'a pas d'influence sur la variation de la fréquence d'oscillation de l'élément vibrant.

La présente invention a alors pour objet un capteur de flux thermique selon la revendication 1.

Les moyens de suspension et d'isolation thermique comportent avantageusement au moins une poutre dimensionnée de sorte que les pertes thermiques à travers les moyens de suspension de l'élément vibrant vers le support soient réduites. Le dimensionnement de la poutre consiste à choisir sa section, sa longueur, sa forme...

Dans un exemple de réalisation, les moyens de suspension et d'isolation thermique comportent au moins deux poutres alignées de part et d'autre de l'élément vibrant ou inclinés l'une par rapport à l'autre et présentant une liaison commune à l'élément vibrant.

De manière très avantageuse, la poutre présente une section comprise entre 10x10 nm$^2$ et 250x250 nm$^2$ et, de préférence égale à 50x50nm$^2$.

La poutre peut présenter une forme non linéaire, avantageusement en serpentin ou avec au moins un rectangle évidé entre deux tronçons.

De préférence, la poutre est en matériau isolant thermique, par exemple en silicium amorphe ou autre.

De manière très avantageuse, les moyens de suspension et d'isolation thermique comportent une zone d'ancrage au support, formée par un matériau nanostructuré, les trous ainsi formés étant rempli ou non.

Les moyens de chauffage sont par exemple des moyens de chauffage par effet Joule.

Dans un exemple de réalisation, les moyens de chauffage sont en contact direct avec l'élément vibrant.

Les moyens de chauffage peuvent être formés par au moins un élément conducteur électrique relié à une source de polarisation et à l'élément vibrant.

Les moyens de détection peuvent être formés par au moins une jauge piézorésistive reliée mécaniquement à l'élément vibrant. De manière avantageuse au moins une jauge piézorésistive forme les moyens de chauffage par effet Joule.

Dans un autre exemple de réalisation, les moyens de chauffage sont situés à distance de l'élément vibrant, le chauffage étant obtenu par conduction à travers l'environnement gazeux entre les moyens de chauffage et l'élément vibrant. Les moyens de chauffage peuvent alors être formés par un fil suspendu.

Les deux éléments vibrants peuvent être disposés de part et d'autre des moyens de chauffage de sorte à assurer une mesure différentielle.

Dans un exemple, l'élément vibrant est un élément vibrant selon des modes de déformation en volume.

Dans un autre exemple, l'élément vibrant est un élément vibrant en rotation.

Les moyens de détection peuvent comporter deux jauges piézorésistives assurant une mesure différentielle. Dans un autre exemple de réalisation, les moyens de détection sont des moyens de détection capacitifs.

Selon l'invention, les moyens d'excitation sont des moyens d'excitation électrostatique.

La présente invention a pour objet un système de mesure de flux thermique comportant une pluralité de capteurs selon la présente invention.

La présente invention a pour objet un système de détermination de la concentration d'un environnement gazeux comportant au moins un capteur de flux thermique selon la présente invention ou un système selon la présente invention, une électronique de traitement des valeurs de tension électrique délivrées par le capteur de flux thermique.

Le au moins un capteur peut être placé dans une boucle électronique de mise en oscillation et de mesure de la fréquence ou de la phase.

La présente invention a pour objet un dispositif d'analyse d'un gaz ou mélange de gaz comportant une colonne de chromatographie en phase gazeuse et au moins un système de détermination de la concentration selon la présente invention, ledit système de détermination étant disposé dans un canal connecté à la sortie de la colonne de chromatographie en phase gazeuse.

## BRÈVE DESCRIPTION DES DESSINS

La présente invention sera mieux comprise à l'aide de la description qui va suivre et des dessins en annexes, sur lesquels :

- la figure 1 est une vue de dessus représentée schématiquement d'un premier mode de réalisation d'un capteur de flux thermique
- les figures 2A et 2B sont des représentations graphiques d'exemples de signaux de résonance électrique de l'élément vibrant obtenus grâce au capteur de la figure 1,
- la figure 3 est une représentation d'un modèle thermique applicable au capteur de la figure 1,
- la figure 4A est une représentation graphique d'une variation de concentration en analyte en fonction du temps,
- les figures 4B et 4C sont des représentations graphiques des variations de température et de fréquence résultant de la variation de concentration de la figure 4A respectivement mesurées pour un capteur de la figure 1,
- la figure 5 est une vue de dessus représentée schématiquement d'une variante de réalisation d'un capteur selon le premier mode de réalisation,
- les figures 6A à 6C sont des variantes de réalisation des moyens de suspension adaptés à la suspension d'un élément vibrant pour un capteur selon l'invention,
- la figure 6D est une vue de dessus représentée schématiquement d'un exemple de réalisation d'un capteur avec des ancrages munis de trous,
- la figure 7 est une vue de dessus représentée schématiquement d'un autre exemple de réalisation d'un capteur selon le premier mode de réalisation, l'élément vibrant vibrant selon des modes de déformation en volume,
- les figures 8A à 8C sont des variantes de réalisation de l'élément vibrant adapté au capteur de la figure 7,
- la figure 9 est une vue de dessus représentée schématiquement d'un capteur selon un deuxième mode de réalisation,
- les figures 10A à 10H sont des représentations schématiques de différentes étapes d'un exemple de procédé de réalisation d'un capteur selon l'invention;
- la figure 11 est une vue de dessus représentée schématiquement d'une variante de réalisation dans lequel les ancrages sont nanostructurés,
- la figure 12 est une vue de dessus représentée schématiquement d'une autre variante de réalisation dans lequel les ancrages isolants et sont recouverts d'une couche conductrice,
- les figures 13A et 13B sont des vues de dessus et de côté d'un exemple de réalisation dans lequel l'élément vibrant est une plaque en rotation,
- la figure 14 représente un organigramme des différentes étapes d'un exemple de procédé de mesure de la variation de fréquence de l'élément vibrant.

## EXPOSÉ DÉTAILLÉ DE MODES DE RÉALISATION PARTICULIERS

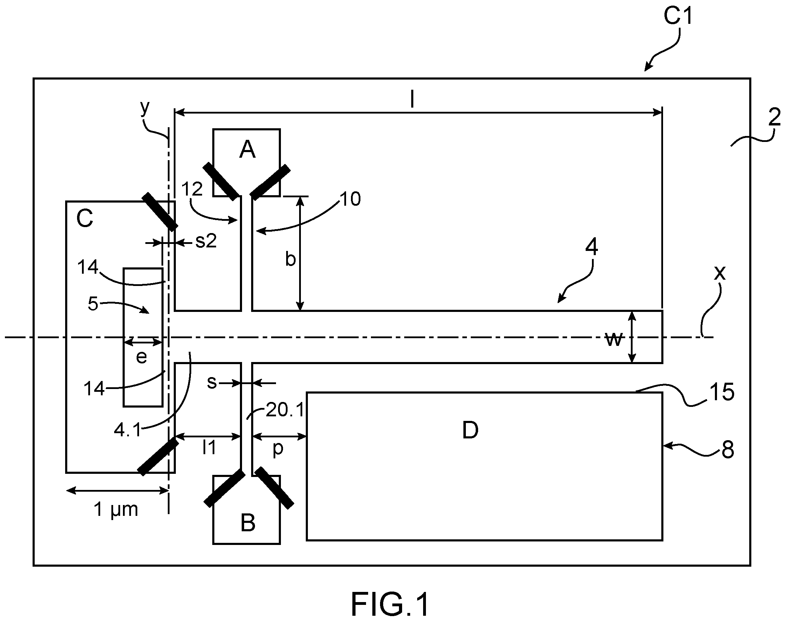

Sur la figure 1, on peut voir une vue de dessus d'un mode de réalisation d'un capteur de flux thermique C1 comportant une partie fixe 2 et une partie mobile 4. La partie fixe 2 est formée par exemple par un support formé par un substrat dans le domaine microélectronique. La partie mobile 4 est formée par un élément apte à osciller par rapport au substrat 2. La partie mobile 4 sera désignée "élément vibrant" dans la suite de la description. L'élément vibrant 4 est formé dans l'exemple représenté par une poutre d'axe longitudinal X reliée à une extrémité longitudinale 4.1 au substrat 2 par des moyens de suspension 5.

Le capteur C1 comporte également des moyens d'excitation 8 de l'élément vibrant 4, des moyens de chauffage 10 de l'élément vibrant 4 et des moyens de détection 12 de la vibration de l'élément vibrant 4.

Les moyens de suspension 5 sont tels qu'ils assurent une isolation thermique de la poutre par rapport au support.

De manière avantageuse, les moyens de suspension présentent une conductance réduite, réduisant ainsi les pertes thermiques de l'élément vibrant 4 vers le substrat 2. Dans l'exemple représenté, les moyens de suspension sont formés par deux poutres 14 alignées d'axe longitudinal Y perpendiculaire à l'axe X de l'élément vibrant 4 et s'étendant de part et d'autre de l'élément vibrant 4 au niveau de son extrémité longitudinale 4.1. Les poutres 14 sont suspendues par leur autre extrémité longitudinale à un plot d'ancrage C pouvant former éventuellement le plot de contact électrique.

Dans l'exemple représenté, le plot d'ancrage C a la forme d'un C.

Les poutres 14 sont de faible section, de manière avantageuse elles sont formées par un nanofil. Les poutres 14 ont une section comprise de préférence entre 10x10 nm$^2$ et 250x250 nm$^2$. Ainsi les pertes thermiques de l'élément vibrant vers le substrat 2 sont limitées par la section des poutres 14.

Il est aussi possible d'utiliser des matériaux isolants thermiquement tels que du silicium amorphe, qui présente une conductivité thermique 4 W.m$^{-1}$.K$^{-1}$ comparée à celle du silicium monocristallin qui est de 148 W.m$^{-1}$.K$^{-1}$, ou un isolant tel du SiN dont la conductivité thermique est 2 W.m$^{-1}$.K$^{-1}$.

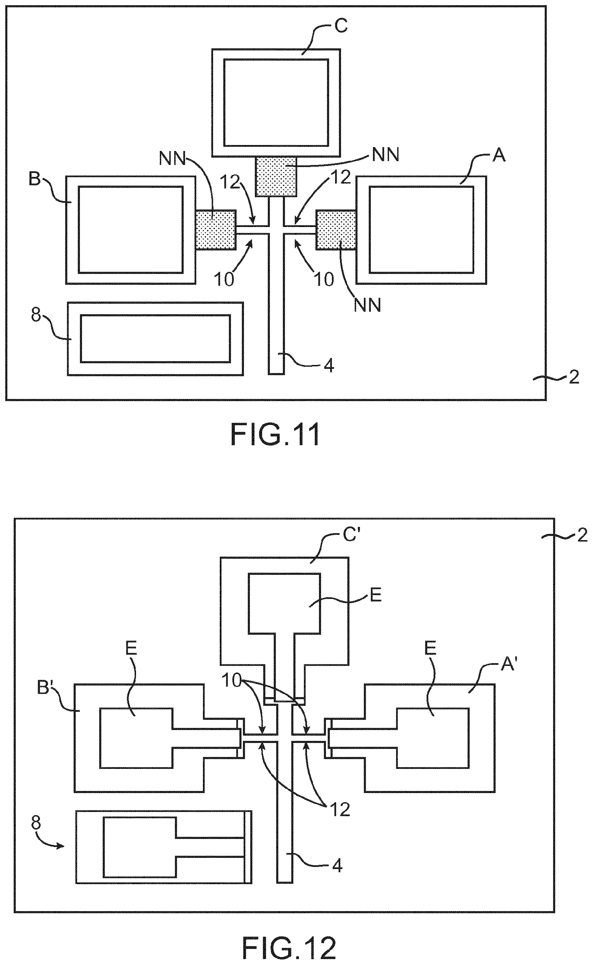

De manière avantageuse il est possible d'améliorer encore l'isolation thermique des suspensions en nanostructurant la partie du support qui sert d'ancrage mécanique aux suspensions. En effet, en faisant par exemple des trous de taille nanométrique, par exemple dans du silicium, de l'ordre de 20 nm de diamètre avec un pas de 15 nm à 20 nm, il est possible de bloquer la diffusion de la chaleur tout en préservant les bonnes propriétés de conduction électriques. Ainsi les encastrements des suspensions deviennent isolants thermiquement mais restent conducteurs pour les électrons (ou les trous). A titre d'exemple, un silicium monocristallin nanostructuré de la sorte exhibe des conductivités thermiques aussi faibles que le silicium amorphe. La figure 11 donne un exemple de tels encastrements NN. Ces moyens isolants sont appelés isolateurs phononiques.

Il peut être envisagé de remplir les trous avec un matériau isolant tel que du SiO$_2$ ou SiOC, voire du SiN... ce qui peut être avantageux sur un plan mécanique en renforçant les ancrages.

En variante, il est aussi possible de réaliser une zone d'encastrement en matériau isolant à la fois thermique et électrique, en utilisant par exemple du SiN ou du SiO$_2$.

Ce type d'isolation est particulièrement adapté au silicium ou SiGe ou Ge.

Les moyens d'excitation 8 sont de type électrostatique et comportent une électrode 15 désignée D fixe sur le substrat et en regard d'une face latérale de l'élément vibrant 4. En appliquant une différence de tension entre l'électrode 15 et l'élément vibrant 4, celui-ci est mis en vibration à sa fréquence de résonance.

L'élément vibrant 4 est alors mis en vibration par les moyens d'excitation de sorte à pivoter autour d'un axe Z perpendiculaire aux axes X et Y.

Dans l'exemple représenté, les moyens de détection 12 de la vibration de l'élément vibrant 4 sont formés par deux jauges de contraintes 20.1, 20.2 disposées de part et d'autre de l'axe longitudinal X suspendues entre l'élément vibrant 4 et des plots d'ancrage A, B formant avantageusement plots de contact. Ainsi, le déplacement en rotation de l'élément vibrant 4 autour de l'axe Z provoque une déformation des jauges 20.1, 20.2, cette déformation étant représentative du déplacement de l'élément vibrant 4.

Les jauges 20.1, 20.2 sont par exemple des jauges piézorésistives. On mesure alors une variation de résistance électrique au sein des jauges 20.1, 20.2 qui est proportionnelle à la contrainte appliquée aux jauges. On polarise les jauges 20.1, 20.2 entre les plots A et B, leur variation de résistance électrique, provoque une variation d'une tension de sortie entre le plot C et la masse.

Dans l'exemple représenté, les jauges forment des moyens de mesure différentiels permettant de s'affranchir des variations extérieures, par exemple des variations lentes de température de l'environnement. En variante, les moyens de détection pourraient ne comporter qu'une seule des deux jauges 20.1, 20.2.

Avantageusement, les jauges sont situées au plus près de l'axe de rotation où les contraintes provoquées par le déplacement de la poutre sont maximales.

Dans l'exemple représenté, les moyens de chauffage 10 sont formés de manière très avantageuse par les jauges 20.1, 20.2 elles-mêmes. L'échauffement de l'élément vibrant 4 est obtenu par effet Joule au sein des jauges et par conduction. On utilise alors la polarisation des jauges utilisée pour la mesure de détection comme moyen de chauffage, ce qui permet de simplifier le capteur.

En variante, les poutres de suspension peuvent servir de moyens de chauffage par effet Joule, les poutres de suspension sont alors conductrices électriques. Afin den réduire les pertes thermiques, les encastrements sont avantageusement nanostructurés

En variante, il est envisageable d'ajouter des moyens de chauffage type nanofils distincts des jauges et des poutres, néanmoins l'ajout de tels nanofils a pour effet de réduire les contraintes dans les jauges et donc le signal mesuré.

La température de chauffage de l'élément vibrant est par exemple comprise entre 300°C et 1000°C.

Les jauges présentent avantageusement une faible section afin de concentrer la contrainte subie et d'augmenter la réponse mécanique. En outre cette faible section permet de limiter les pertes thermiques de l'élément vibrant 4 vers le substrat 2. De préférence, les jauges ont une section comprise entre 10x10 nm$^2$ et 250x250 nm$^2$ pour des longueurs entre 100nm et 500nm

Il est également envisageable d'augmenter l'isolation thermique en nanostructurant la zone d'encastrement des jauges de la même manière que les plots d'encastrement des moyens de suspension décrit sur la figure 11.

Sur la figure 12 représente un exemple de réalisation d'un capteur dans lequel les encastrements A', B', C' sont réalisés en un matériau isolant thermique et électrique, tel que du SiN ou du SiO$_2$. Dans cette variante, les encastrements A', B', C' sont recouverts d'une couche fine de matériau conducteur électrique E, de préférence un métal, dont l'épaisseur reste très faible par exemple inférieure à 10 nm. De préférence ce métal sera du TiN ou de l'AlSi. Le flux thermique vers le support est donc limité.

De préférence on réalise à la fois les suspensions, les jauges dans le cas du mode de détection piézorésistif et l'élément vibrant en silicium monocristallin. On privilégie alors des nanofils pour réaliser les jauges et les suspensions et /ou un ancrage isolant de type ancrage nano structuré.

Des exemples de dimensions caractéristiques du capteur sont donnés dans le tableau ci-dessous

- w est la largeur de l'élément vibrant 4
- l est la longueur de l'élément vibrant 4
- l1 est la distance entre les jauges 20.1, 20.2 et la poutre 14.
- b est la longueur des jauges 20.1, 20.2, de l'ordre de 200 nm à 400 nm.
- s est la largeur des jauges 20.1, 20.2, comprise typiquement entre 80 nm et 160 nm.
- s2 est l'épaisseur des poutres 14.
- e est la distance entre le plot d'ancrage C et le bord latéral en regard des poutres 14.
- p est la distance entre la jauge 20.1 et l'électrode D,
- g est la distance entre l'électrode et la face latérale en regard de l'élément vibrant 4.

| w | L | l1 | b | s | s2 | e | P | g |
|---|---|---|---|---|---|---|---|---|
| 300 nm | 3,2 $\mu$m | 480 nm | 300 nm | 100 **nm** | 120 **nm** | 250 nm | 250 nm | 150 nm |
| 300 nm | 3,2 $\mu$m | 480 nm | 600 nm | 100 **nm** | 120 **nm** | 250 nm | 250 nm | 150 nm |
| 300 nm | 7 $\mu$m | 1,05 $\mu$m | 600 nm | 100 **nm** | 150 **nm** | 250 nm | 250 nm | 150 nm |

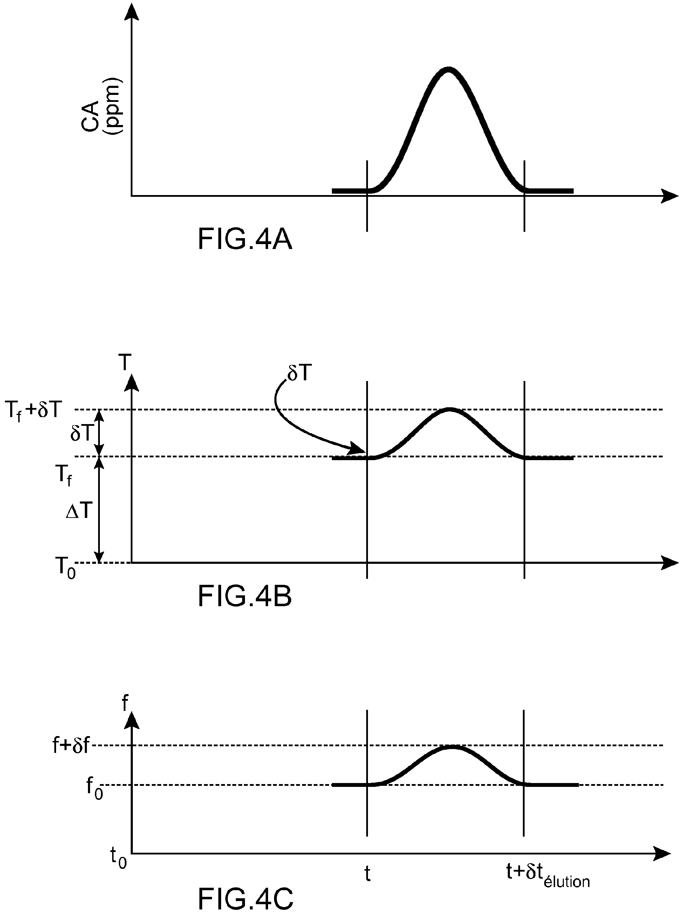

Nous allons décrire le principe de fonctionnement du capteur de la figure 1 dans une application à la détermination de la composition d'un environnent gazeux en relation avec les représentations graphiques des figures 4A à 4C. L'environnement gazeux comporte un gaz porteur, par exemple de l'hélium ou de l'air sec, et des analytes, c'est la concentration en analytes que l'on souhaite déterminer.

La figure 4A est une représentation graphique d'une variation de concentration CA en analyte en ppm du gaz à analyser en fonction du temps,

La figure 4B est une représentation graphique de la variation de la température T la variation de concentration de la figure 4A en fonction du temps de l'élément vibrant 4.

La figure 4C est une représentation graphique de la variation de fréquence de résonance f de l'élément vibrant 4 résultant de la variation de concentration de la figure 4A.

A l'état initial $t_0$ l'élément vibrant 4 est chauffé par effet Joule à la température $T_0+\Delta T$ qui est la température de fonctionnement $T_f$, par l'intermédiaire des jauges 20.1, 20.2. $\Delta T$ est choisi pour minimiser les effets de la conduction autre que celle qui se produit dans le gaz qui est le gaz porteur. La température de fonctionnement est choisie afin d'optimiser le rapport signal à bruit et le coefficient $\alpha$ décrit ci-dessous.

A un instant t, un analyte mélangé au gaz porteur arrive sur le capteur modifiant ainsi les propriétés de conduction du gaz environnant désignée $G_g$.

Les constantes de temps fluidique considérées, i.e. le front du pic d'élution et la durée de ce pique de l'ordre de 100 ms, sont grandes devant la réponse mécanique qui est par exemple comprise entre 0,1 $\mu$s et 1 $\mu$s et la réponse thermique qui est par exemple inférieure à 500 ns. Tant que l'analyte est présent dans le gaz porteur, la conduction passe de $G_g$ à $G_g+\delta G$ ce qui provoque une variation de température $\delta T$ par rapport à la température de fonctionnement $T_f$. Cette variation de température induit un décalage en fréquence. Par ailleurs, la contrainte variant avec les vibrations, la variation de fréquence des vibrations provoque une variation des contraintes, et la fréquence du signal électrique obtenu par effet piézorésistif varie aussi.

Lorsque l'analyte n'est plus présent dans le gaz porteur à la fin du pic d'élution, au bout d'un temps $\delta t_{\text{élution}}$ la température revient à $T_f$. Le capteur est prêt à détecter un autre pic d'analyte.

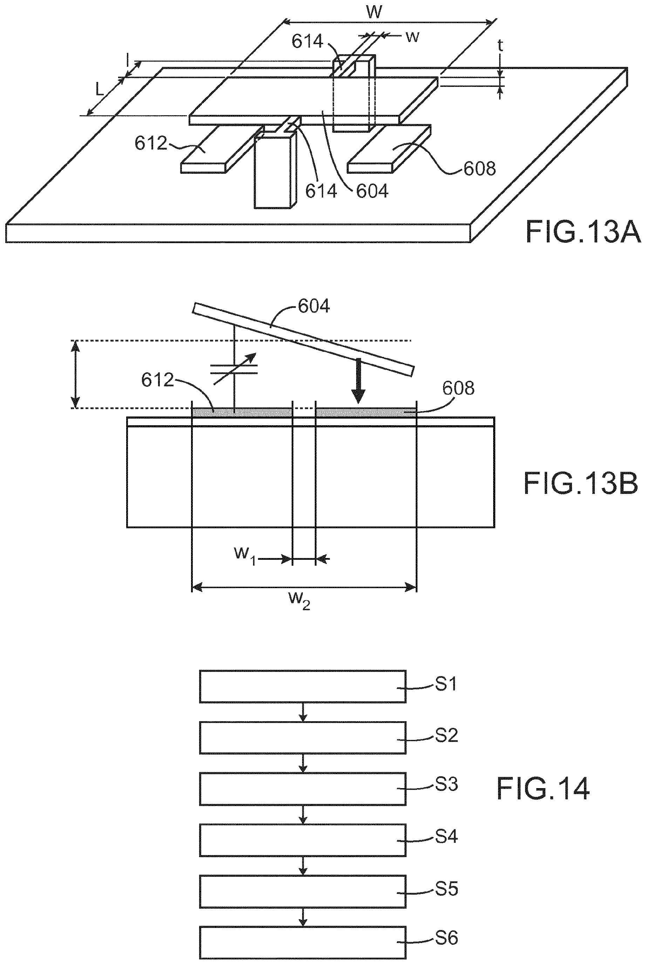

Sur la figure 14 est représenté un organigramme des différentes étapes d'un exemple de procédé de mesure de la variation de fréquence de l'élément vibrant.

Lorsqu'un pic d'élution, correspondant à un analyte gazeux ayant une certaine largeur temporelle, par exemple 100 ms à quelques secondes de durée et une certaine largeur spatiale par exemple 1 mm, vient autour de la structure vibrante (étape S1) l'équilibre thermique du système est alors modifié (étape S2). La diffusion de chaleur à travers le gaz est alors modifiée : l'environnement gazeux étant en général moins conducteur thermique que le gaz porteur (en l'absence d'analyte), le système vibrant se réchauffe (étape S3). En effet l'élément vibrant étant isolé thermiquement, i.e. il a peu d'échange avec le support en silicium, la température de l'élément vibrant est principalement fixée par le gaz environnant. Lorsque le matériau de l'élément vibrant s'échauffe ses propriétés mécaniques (notamment le module d'Young, rapport de Poisson, contraintes) sont modifiées (étape S4) provoquant un décalage de la fréquence de résonance du système mécanique (étape S5). Le système mécanique ayant une réponse temporelle largement plus rapide que celle du pic d'élution (1 μs comparé à 1 s) et plus rapide que la constante thermique du système gaz/surface de silicium (de l'ordre de 100 μs), la fréquence est une image parfaite du pic d'élution (étape S6). Pour réaliser la mesure, le système vibrant est intégré dans une boucle de mesure de la fréquence. Celle-ci peut être une PLL (Boucle à verrouillage de phase) ou une boucle auto-oscillante. La fréquence est alors suivie en temps réel.

L'élément vibrant 4 est excité par une force électrostatique à sa fréquence de résonance $\omega_0$. La force $F_g$ agissant sur les jauges peut s'exprimer comme suit (dans le domaine fréquentiel) en considérant un modèle équivalent masse/ressort :

$$F_g(\omega) = \beta \frac{\omega_0^2}{\omega_0^2 - \omega^2 + j\omega\omega_0 / Q} F_{el}(\omega)$$

$\beta$, $\omega$, Q and $F_{el}(\omega)$ sont le facteur d'amplification du bras de levier produit par la poutre, la fréquence angulaire (rad/s), le facteur de qualité, et la force électrostatique respectivement appliquée entre l'électrode 15 et l'élément vibrant 4.

La force électrostatique est générée par une tension RF désignée $V_{drive}(\omega)$. On peut également prévoir d'ajouter une tension continue $V_{DC}$. Dans ce cas, puisque la force est proportionnelle à la tension au carré, le signal d'excitation alternatif présente une fréquence égale à la fréquence de résonance de la poutre. Si le signal d'excitation n'a qu'une composante alternative, sa fréquence est la fréquence de résonance divisée par 2. Les deux cas sont possibles. Une excitation à 2w présente l'avantage d'avoir moins de fond continu.

L'une des jauges 20.1, 20.2 est comprimée et l'autre jauge 20.2, 20.1 est simultanément étirée sous la contrainte $F_g/s$, s étant la section des jauges).

La variation de résistance $\Delta R_{par}$ effet piézorésistif dans les jauges 20.1, 20.2 s'exprime donc comme suit :

$$\frac{\Delta R(\omega)}{R} = \gamma \frac{F_g(\omega)}{2 \cdot s \cdot E}$$

$\gamma$ and E sont les facteurs de jauge et le module d'Young des jauges. La valeur de G dépend du matériau utilisé pour les jauges, par exemple du type de semi-conducteur et de son dopage. G est généralement compris entre 10 et 100.

La tension de sortie $V_S$ sur C par rapport à la masse s'exprime :

$$V_s(\omega) \propto \frac{\gamma\beta V_{bias} V_{DC} V_{drive}(\omega)}{2 \cdot s \cdot E} \frac{Q\omega_0^2}{\sqrt{(\omega_0^2 - \omega^2)^2 + \omega^2\omega_0^2}}$$

$V_S$ est proportionnelle à la tension de bias et à la tension de RF. Si la tension DC n'est pas utilisée, $V_S$ sera proportionnelle au carré de la tension RF.

De manière préférentielle, la tension de bias est une tension alternative permettant de faire une mesure hétérodyne, i.e. permettant d'avoir une fréquence de détection différente de la fréquence d'excitation, ce qui peut permettre de travailler à des fréquences d'excitation élevées.

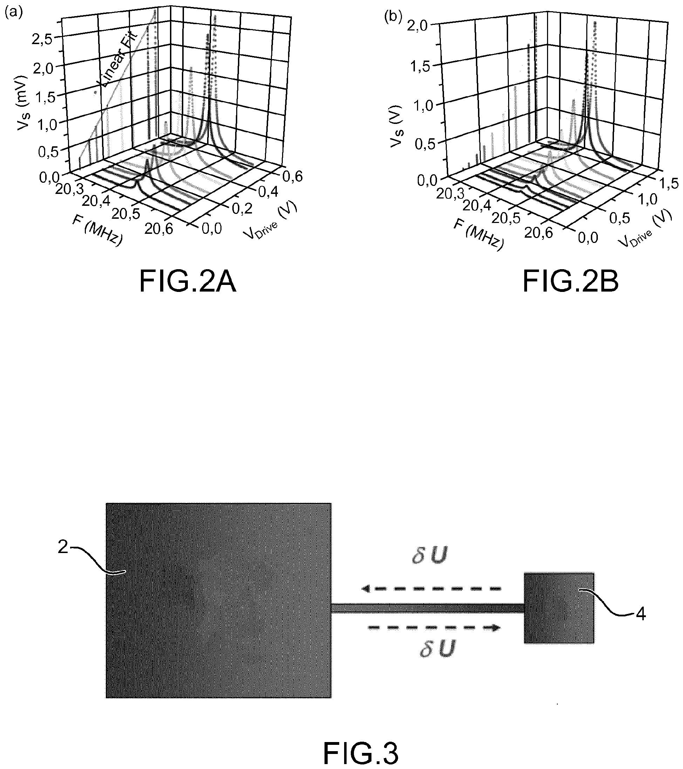

L'élément vibrant 4 est mis en résonance et la variation de la fréquence de résonance de l'élément vibrant 4 est suivi. Sur les figures 2A et 2B, on peut voir des exemples de la variation de la tension Vs en fonction de la tension de bias et la tension en fonction de tension $V_{drive}(\omega)$ respectivement à la fréquence de résonance de l'élément vibrant.

L'effet de la température de l'élément vibrant sur sa fréquence de résonance va maintenant être décrit.

Considérons la variation de la fréquence de résonance en fonction de la température. Il est connu que, pour un point de fonctionnement ($T_0$) donné, la fréquence de résonance des matériaux, par exemple pour les matériaux semi-conducteurs, varie proportionnellement avec la température : cette variation s'écrit:

$$\omega_0(T) = \omega_0(T_0)(1 + \alpha\Delta T)$$

$$\Re \equiv \alpha = \frac{1}{\omega_0(T_0)} \frac{d\omega}{dT}$$

$\alpha$ est le coefficient thermique, $\Delta T$ est la variation de température par rapport à $T_0$. Dans le cas du silicium $\alpha$ est de l'ordre de - 100ppm à température de 300 K. $\alpha$ constitue la réponse thermique $\mathfrak{R}$ du système, la fréquence étant le paramètre de mesure.

Il existe des échanges thermiques entre l'élément vibrant 4 et son environnement.

On souhaite connaître la température de l'élément vibrant à tout moment en considérant que celui-ci échange de la chaleur à travers le gaz environnant et avec les supports mécaniques à travers les poutres 14 et les jauges.

En première approximation un modèle 1D de type RC thermique schématisé sur la figure 3 est utilisé. Etant données les dimensions considérées les phénomènes de convexion sur l'élément vibrant 4 en présence ou non de capot sur le capteur sont négligeables. Seule la conduction sera considérée.

$\delta U$ représente l'échange d'énergie interne entre le réservoir thermique possédant une température fixée, formant thermostat, formé par le substrat 2 et la capacité thermique de l'élément vibrant 4 désignée C.

L'équation de diffusion de la chaleur du système s'écrit comme suit :

$$C\frac{d\Delta T}{dt}+G\Delta T = P(t)$$

G est la conductance via le gaz et les connexions mécaniques solides formées par les jauges 20.1, 20.2 et les poutres 14. P(t) est la puissance thermique apportée sur le système. $\Delta T$ est l'élévation de température de l'élément vibrant 4.

Dans le mode de réalisation de la figure 1, la puissance thermique est générée à travers les jauges 20.1, 20.2 par effet joule. La tension de lecture $V_{bias}$ qui est généralement alternative peut servir à produire la chaleur nécessaire à l'échauffement du capteur par auto-échauffement. On peut superposer à cette tension de lecture $V_{bias}$ une tension continue dédiée $V_{chauffe}$. En précisant les notations et en détaillant les expressions l'équation précédente devient :

$$C\frac{d\Delta T}{dt}+(G_g + G_{jauges} + G_{encastrements})\Delta T = \frac{V(t)^2}{R_{jauges}}$$

avec $V(t) = V_{bias}+V_{chauffe}$ ou $V(t)=V_{bias}$. R est la résistance électrique des jauges.

Considérons que $V(t) = V_0 H(t\text{-}t_0)$ ou $H(t\text{-}t_0)$ est la fonction de Heaviside. On peut résoudre cette équation en considérant d'une part la partie statique et d'autre part le transitoire. Le transitoire correspond à une exponentielle croissante de constante de temps :

$$\tau_{th} = C\big/(G_g + G_{jauges} + G_{encastrement})\ .$$

L'élévation de température $\Delta T$ à l'état stable correspond à :

$$\Delta T = V_0^2\big/R_{jauges}(G_g + G_{jauges} + G_{encastrement})\ .$$

$V_0$ est la tension RMS du signal si celui-ci est alternatif.

Il en résulte que la réponse du système en fréquence à l'incrément de température est donc (en Hz ou rad.s$^{-1}$) :

$$\Delta\omega = \frac{\alpha\omega_0 V_0^2}{R_{jauges}(G_g + G_{jauges} + G_{encastrements})}$$

En choisissant des jauges et des encastrements présentant des conductivités thermiques très faibles, la réponse thermique du système en fréquence $\Delta\omega$ est maximisée. De plus, celle relation montre que, de préférence, on choisit les conductivités thermiques des jauges et des encastrements de celles-ci les plus faibles possibles afin de voir au mieux l'effet de la variation de la conductivité thermique du gaz. A partir de cette variation de fréquence de résonance, on peut remonter à la variation de température de l'élément vibrant et en déduire la composition du pic.

Pour suivre la variation de fréquence de l'élément vibrant 4, le capteur est placé dans une boucle électronique de mise en oscillation et de mesure de la fréquence ou de la phase. Le capteur peut être placé dans une boucle à verrouillage de phase (PLL) ou dans une boucle auto-oscillante. La résolution de l'ensemble formé par le capteur et l'électronique est donc fonction de sa stabilité en fréquence, c'est-à-dire la fluctuation de la fréquence $\sigma_\omega$ dans le temps autour de la fréquence nominale :

$$\sigma_\omega^2 = \frac{S_\omega(\omega = \omega_0)}{\tau}$$

La résolution fréquentielle, qui est la plus petite fréquence mesurable fixée par les bruits intrinsèques au système, et la résolution en température sont respectivement:

$$\delta\omega = \sqrt{\frac{\left(\dfrac{\omega_0^2}{4Q^2}\dfrac{1}{SNR^2} + \dfrac{\omega_0 \alpha k_B \cdot T^2}{(G_{jauges} + G_{encastrements})}\right)}{\tau}}$$

$$\delta T = \Re^{-1}\sqrt{\frac{\left(\dfrac{\omega_0^2}{4Q^2}\dfrac{1}{SNR^2} + \dfrac{\omega_0 \alpha k_B \cdot T^2}{(G_{jauges} + G_{encastrements})}\right)}{\tau}}$$

$$\delta T = \frac{\sqrt{\left(\dfrac{\omega_0^2}{4Q^2}\dfrac{1}{SNR^2} + \dfrac{\omega_0 \alpha k_B \cdot T^2}{(G_{jauges} + G_{encastrements})}\right)}}{\alpha\sqrt{\tau}}$$

Ces résolutions sont optimisées avec la température de travail, les tensions de lecture et d'actionnement du système, le choix du matériau (en particulier les paramètres : le coefficient en température $\alpha$, la résistance électrique R et le module d'Young E varient différemment selon le matériau choisi) et la morphologie du capteur. Comme décrit ci-dessus, la conductance thermique est définie en isolant la poutre du support et en choisissant un matériau adéquat.

Différentes méthodes de transduction peuvent être choisies de façon minimiser le bruit, par exemple une mesure capacitive au lieu d'utiliser des jauges piézorésistives pour ce type de jauge vibrante.

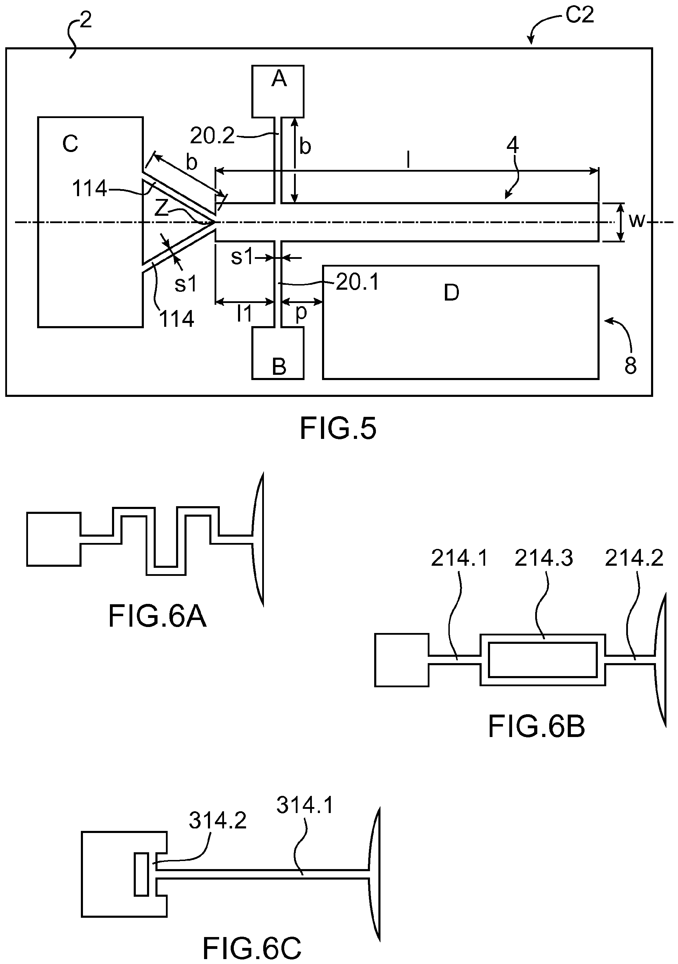

Sur la figure 5, on peut voir une variante de réalisation d'un capteur selon le premier mode de réalisation. Celui-ci C2 diffère du capteur de la figure 1 par la forme des moyens de suspension.

Les mêmes références seront utilisées pour décrire les éléments ayant la même fonction et ayant une structure proche ou similaire.

Le capteur de la figure 5 comporte un élément vibrant 4 par rapport à un substrat 2 par des moyens de suspension formés par deux poutres 114 reliées par une première extrémité longitudinale au plot d'ancrage C et par une deuxième extrémité longitudinale à l'extrémité longitudinale 4.1 de l'élément mobile. Les poutres 114 diffèrent des poutres 14 en ce que leur axes longitudinal est inclinée par rapport à l'axe X et sont concourantes au niveau de leur deuxième extrémité longitudinale à l'axe Z. Dans l'exemple représenté, les poutres 114 sont inclinées d'un angle de 45° par rapport à l'axe longitudinal X.

Dans les exemples des figures 1 et 5, les moyens de suspension comportent deux poutres. On peut envisager qu'ils comportent une poutre ou plus de deux poutres.

Sur les figures 6A à 6C, on peut voir d'autres exemples de réalisation de moyens de suspension adaptés pour suspendre l'élément vibrant par rapport au substrat.

Sur la figure 6A, la poutre présente une forme en créneau. Sur la figure 6B, la poutre comporte deux portions droites 214.1 reliés par une portion en forme de cadre 214.2. Cette réalisation permet de réduire les pertes thermiques de manière très efficace et d'encaisser les effets de dilatation.

Sur la figure 6C, les moyens de suspension comportent une poutre droite 314.1 raccordée au substrat 2 par une poutre suspendue 314.2 perpendiculaire à la poutre droite 314.1

Il sera compris que les différents exemples de moyens de suspension peuvent être combinés, par exemple la poutre en créneau 6A ou celle de la figure 6B pourrait être reliée au substrat par une poutre transversale de la figure 6C.

En outre, les moyens de suspension peuvent être formés par plusieurs poutres des figures 6A à 6C.

Les éléments de suspension 14 (Figure 1) 114 (Figure 5) sont des petites poutres faisant partie de l'ancrage.

Ils sont de type nanofils de largeur sensiblement égale à celle des jauges (par exemple de l'ordre de 100 nm) inférieure à la largeur de la poutre suspendue formant le capteur vibrant.

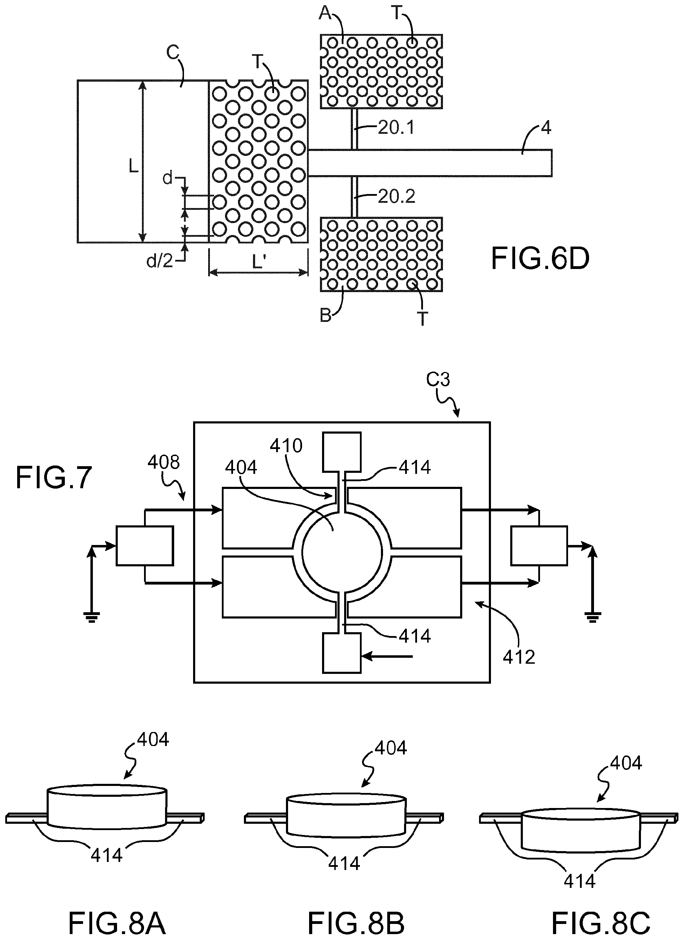

Dans le cas où l'élément vibrant est formé par un disque ou une plaque, les ancrages peuvent être formés par des poutres ou assemblage de poutres (figures 6A à 6B et figure 7). Leurs dimensions sont de préférence inférieures à 500 nm de large pour des longueurs supérieures à 1μm.

Sur la figure 6D, on peut voir un exemple d'ancrages des éléments de suspension d'un élément vibrant sous forme de plague/ disque ou de poutre. Les ancrages A, B et C sont munis de trous 15 permettant d'améliorer encore l'isolation thermique. Leur diamètre est par exemple de l'ordre ou inférieur à 250 nm et peut atteindre 10 nm pour une distance bord à bord de l'ordre 100 nm. les ancrages A et B comportent des trous sur toute leur surface et l'ancrage C comporte des trous sur une partie de sa surface du côté de la connexion à la poutre suspendue. Cette partie présente une largeur L', et les trous ont un diamètre d.

Dans le tableau ci-dessous sont rassemblés des exemples de dimensions pour le diamètre d des trous et la largeur L' de partie structurée de l'ancrage C, pour une largeur L de l'ancrage C de 10 μm.

| L' (μm) | d (nm) |
|---------|--------|
| 20      | 50     |
| 20      | 100    |
| 50      | 200    |
| 50      | 250    |
| 100     | 250    |

Dans l'exemple des figures 1 et 5, l'élément vibrant est formé par une poutre. Selon un autre exemple de réalisation, les éléments suspendus formant résonateurs peuvent être des plaques ou des disques vibrant selon des modes de déformation en volume, par exemple en mode extensionnel.

Sur la figure 7, on peut voir un tel exemple de réalisation. L'élément vibrant 404 du capteur C3 est formé par un disque suspendu par deux poutres droites 414 diamétralement opposées. Le capteur comporte des moyens d'excitation 408 de type électrostatique formés par des électrodes portées par le substrat 2 en regard d'un bord du disque 404 d'un côté des poutres de suspension 414. Ces moyens d'excitation génèrent un mouvement dans le plan du substrat. Le capteur comporte également des moyens de détection 412 de type capacitif formés par des électrodes portées par le substrat en regard de l'autre bord du disque 404 de l'autre côté des poutres de suspension 414. Les moyens de détection capacitifs 412 présentent l'avantage d'éviter d'avoir une liaison mécanique entre l'élément vibrant 404 et le support 2, ce qui réduit les pertes thermiques. De plus cela permet d'avoir moins de bruit à la détection, en revanche les éléments sont un peu plus épais, de l'ordre de μs à 10 μs, et présentent une inertie thermique plus grande.

Comme cela est représenté sur les figures 8A à 8C, l'épaisseur des éléments vibrants peuvent être différente de celles des poutres de suspension ce qui permet de réaliser des poutres de suspension présentant une faible section pour réduire les pertes thermiques. A titre d'exemple, les éléments vibrants peuvent avoir une épaisseur de 100 nm à 5 μm et les poutres peuvent avoir une épaisseur de 10 nm à 100nm. Le diamètre du disque peut être compris entre 1 μm et 100 μm.

Dans l'exemple représenté, l'élément vibrant formant résonateur a la forme d'un disque mais d'autres formes sont envisageables, par exemple une forme carrée, une forme rectangulaire, ou une forme de cadre annulaire ou rectangulaire ou encore carré.

Les moyens de chauffage 410 sont avantageusement formés par les poutres de suspension 414 qui sont polarisées et assurent un chauffage par effet Joule.

La mise en œuvre de moyens de détection capacitifs présente en outre l'avantage de minimiser les bruits de Johnson qui ne seront alors issus que des connections électriques. Ainsi une poutre vibrante avec un actionnement électrostatique et une détection capacitive est aussi envisageable en utilisant la première électrode pour l'actionnement et la seconde électrode pour la détection par exemple.

En variante, on peut envisager des moyens d'excitation tels qu'ils génèrent un mouvement de l'élément suspendu perpendiculaire au plan du substrat, dit mouvement hors plan. Sur les figures 13A et 13B, on peut voir un exemple de réalisation d'un tel capteur. Dans cet exemple, l'élément vibrant 604 est une plaque mobile en rotation autour d'un axe de rotation formé par deux poutres de suspension 614 sollicitées en torsion, formées par des nanofils. Une première électrode 608 est placée en-dessous de l'élément vibrant sur le substrat à de sorte à exciter l'élément vibrant. Une deuxième électrode 612 est prévue à côté de la première électrode pour assurer la détection capacitive. En variante, les deux électrodes participent à l'excitation, et à des intervalles de temps donnés une détection est réalisée par l'une ou les deux électrodes.

Les électrodes sont formées soit par un dépôt métallique soit par un dopage plus fort localisé. La plaque vibrante peut être en silicium monocristallin. Les électrodes sont par exemple en AlSi dopé.

Les encastrements peuvent être réalisés dans le même matériau ou dans un autre matériau, par exemple en un isolant thermique comme du SiN ; dans ce cas une couche métallique fine est déposée sur le ou les encastrements pour assurer le contact électrique tout en minimisant les pertes thermiques - Les encastrements peuvent être nano structurés afin d'assurer une isolation thermique améliorée comme décrit ci-dessus.

A titre d'exemple, la structure de la figure 11 peut présenter les dimensions suivantes:

- L étant la largeur de la plaque vibrante;
- W étant la longueur de la plaque vibrante;
- l étant la longueur des poutres de suspension;
- t étant l'épaisseur de la plaque vibrante et des jauges;
- w étant la largeur des poutres de suspension;
- w2 étant la distance séparant les deux faces opposées des électrodes;
- w1 étant la distance séparant les deux faces en regard des électrodes;
- Gap étant la distance entre l'axe de rotation et la face libre des électrodes en regard de la plaque vibrante.

| Paramètres | $L$ | $W$ | $l$ | $W$ | $W_1$ | $W_2$ | $L_{elec}$ | $t$ | $Gap$ |
|---|---|---|---|---|---|---|---|---|---|
| Valeurs typiques | $12\mu m$ | $12\mu m$ | $1\mu m$ | 50nm | w | 0.8W | L | 50nm | $\lambda/4$ |

La tension d'excitation et la tension de polarisation pour le chauffage appliquées sont de l'ordre de 1V à 20V. Il s'agit d'une tension continue qui peut permettre à la fois l'excitation et le chauffage. Ou alors, on peut réaliser un actionnement à fréquence w/2, sans application de tension continue et dans ce cas cette tension n'assure que le chauffage. Si le chauffage et l'excitation nécessitent une tension du même ordre de grandeur cette tension peut être utilisée pour les deux, sinon on réalise un actionnement à w/2 et la tension continue est efficace uniquement pour le chauffage.

Les poutres de suspension décrites ci-dessus en relation avec les figures 6A à 6C peuvent être mises en œuvre pour suspendre le résonateur de la figure 7.

En variante, on peut envisager un capteur mettant en œuvre un résonateur formé d'un élément vibrant selon des modes de déformation de volume comportant des moyens de détection de type piézorésistif. Pour cela, on détecte la contrainte appliquée sur les poutres de suspension dont au moins une et avantageusement deux forment également des jauges de contrainte. Le fonctionnement est alors similaire à celui de la figure 1. Les jauges forment également les moyens de chauffage. En variante, on peut prévoir des poutres de suspension dédiées au chauffage et d'autres à la détection piézorésistive.

Ce mode de réalisation mettant en œuvre un élément vibrant sous forme de plaque présente l'avantage d'offrir une grande surface d'échange thermique avec l'environnement gazeux.

Selon un deuxième mode de réalisation d'un capteur selon l'invention, les moyens de chauffage sont situés à distance de l'élément vibrant et le chauffage a lieu par conduction à travers l'environnement gazeux.

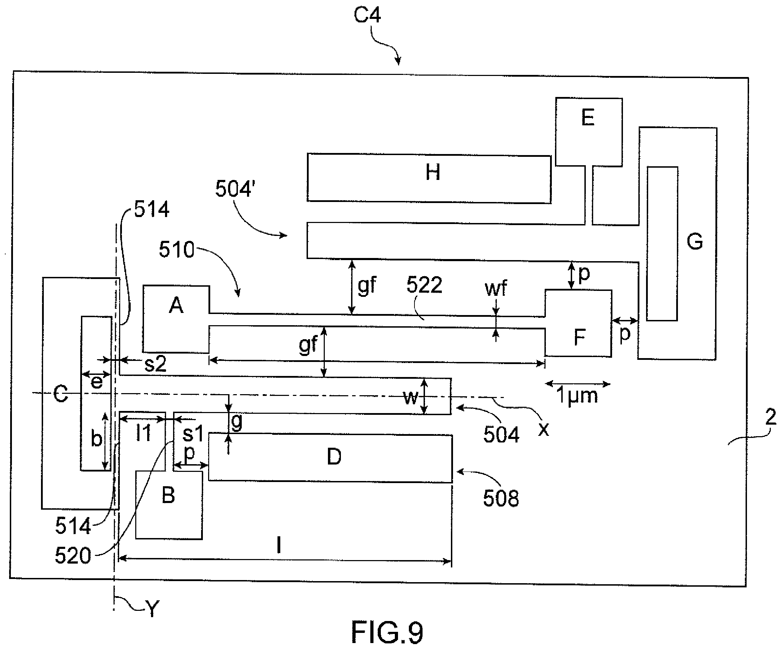

Sur la figure 9, on peut voir un exemple de réalisation d'un capteur selon le deuxième mode de réalisation, ce capteur C4 présente en outre la particularité de comporter deux éléments suspendus 504, 504[1].

Nous décrirons en détail l'élément vibrant 504 et les moyens qui lui sont associés, cette description s'applique également à l'élément vibrant 504'.

L'élément vibrant 504 et les moyens associés sont proches de ceux du capteur de la figure 1. L'élément vibrant 504 est formé par une poutre d'axe longitudinal X suspendue par une des ses extrémités au substrat par deux poutres 514 alignées le long d'un axe Y perpendiculaire à l'axe X. Des moyens d'excitation de type électrostatique 508 sont prévus, ils comportent une électrode en regard d'un bord latéral de l'élément vibrant 504. Les moyens de détection sont formés par une jauge piézoélectrique 520 suspendue entre l'élément vibrant et un plot B et s'étendant le long d'un axe perpendiculaire Y à l'axe longitudinal X.

Le capteur C4 comporte des moyens de chauffage 510 formés par un fil suspendu 522 entre deux plots d'ancrage formant avantageusement plots de contact, le fil 522 est situé à une distance gf de l'élément vibrant 504. De préférence, le fil 522 est parallèle à l'élément vibrant 504 de sorte à assurer un chauffage homogène de l'élément vibrant 504 sur toute sa longueur.

Il sera compris que le fil suspendu 522 peut ne pas être linéaire, mais présenter des formes adaptées à l'encombrement du capteur, par exemple en zigzag ou autre, telles que celles représentées sur les figures 6A à 6C.

L'élément vibrant 504' est disposé à l'opposé de l'élément vibrant 504 par rapport au fil 522 et s'étend selon un axe longitudinal parallèle à l'axe X. De préférence la même distance sépare l'élément vibrant 504' du fil 522 et l'élément vibrant 504 du fil 522 de sorte à permettre une mesure différentielle et à simplifier les mesures.

Comme pour le premier mode de réalisation, les moyens de suspension et les jauges présentent des sections faibles afin de réduire les pertes thermiques des éléments suspendus 504, 504' vers le substrat.

Le fonctionnement du capteur C4 est similaire à celui du capteur C1 sauf pour chauffage des éléments suspendus. En effet dans ce mode de réalisation, le chauffage des éléments suspendus se fait par propagation de la chaleur entre le fil 522 et le les éléments suspendus 504, 504' principalement par conduction thermique à travers le gaz à analyser.

Dans le tableau ci-dessous est donné un exemple de dimensionnement du capteur C4.

- w est la largeur des éléments suspendus 504, 504' ;
- l est la longueur des éléments suspendus 504, 504' ;
- l1 est la distance entre la jauge 520, et la poutre 514 ;
- b est la longueur de la poutre 514, qui est aussi la longueur de la jauge 520 ;
- s1 est la section de la jauge 520 ;
- s2 est la section des poutres 514 ;
- e est la distance entre le plot d'ancrage C et le bord latéral en regard des poutres 514.
- p est la distance entre la jauge 20.1 et l'électrode D,
- g est la distance entre l'électrode et la face latérale en regard de l'élément vibrant 4 ;
- gf est la distance entre le fil 522 et la face latérale en regard de l'élément vibrant 504 ; gf peut être compris entre 100nm et 1 $\mu$m en fonction des encombrements des plots;
- lf est la longueur du fil 522 ;
- wf est la largeur du fil 522 ;

| w | l | l1 | b | s1 | s2 | e | p | gf | lf | wf | gf | g |
|---|---|----|----|-----|-----|----|----|----|----|----|----|----|
| 300 nm | 3,2 $\mu$m | 480 nm | 300 nm | 100 nm$^2$ | 120 nm$^2$ | 250 nm | 250 nm | 750 nm | 3,2 $\mu$m | 100 nm | 200 nm | 150 nm |

Ce mode de réalisation peut présenter une plus grande efficacité de détection, puisque les propriétés du gaz participent dans le chauffage des éléments suspendus. Les résolutions fréquentielles et les résolutions en température peuvent être calculées comme pour le capteur C1.

L'exemple de réalisation de la figure 9 mettant en œuvre deux éléments suspendus présente l'avantage d'avoir accès à une mesure différentielle permettant de faciliter la lecture puisque l'on supprime l'effet du fond continu résiduel, tel que les variations lente de température distincte de celles dues aux moyens de chauffage et aux échanges thermiques avec l'environnement gazeux.

On peut également réaliser un capteur C4 selon le deuxième mode de réalisation ne comportant qu'un seul élément vibrant. Mais ce capteur ne permet pas de réaliser de mesure différentielle.

En variante, on peut prévoir deux jauges par élément vibrant.

Des éléments vibrants selon des modes de déformation peuvent être mis en œuvre dans la structure du capteur selon le deuxième mode de réalisation. Cette réalisation présenterait néanmoins un encombrement plus important que le capteur de la figure 9.

En multipliant les éléments vibrants, on peut augmenter la résolution du capteur. Cette méthode consiste à effectuer un moyennage spatial du bruit sur un grand nombre d'échantillons réduisant de fait le bruit d'un facteur $\sqrt{n}$.

Un tel dispositif de mesure comporte n capteurs, on obtient ainsi un dispositif de mesure dont la résolution est augmentée. Les capteurs sont associés par des connexions croisées, mettant de préférence en œuvre au moins deux niveaux de métallisation.

En considérant la même tension d'actionnement et le même courant de lecture dans chacun des capteurs, la mise en parallèle de n capteurs fréquentiels permet de réduire les bruits de Johnson et de Flicker. Les matrices de capteurs ainsi réalisées peuvent être adressées collectivement, i.e. en utilisant une entrée et une sortie pour un réseau, ou adressées individuellement, i.e. chaque capteur possédant son actionnement et sa lecture.

La structure suspendue peut être réalisée dans différents matériaux, par exemple en silicium dopé N ou P, en Ge, SiGe. Avantageusement du silicium poreux pourrait être utilisé afin d'augmenter la surface d'échange thermique de l'élément vibrant. Il pourrait en particulier constituer la seconde couche épaisse pour les structures présentant deux épaisseurs.

La structure suspendue peut aussi être recouverte d'un métal (Al, AlSi, TiN par exemple).

Un capteur selon l'invention avec une structure suspendue dans laquelle l'élément vibrant présente une épaisseur supérieure à celle de jauges et des poutres de suspension peut par exemple être réalisé suivant un procédé similaire à celui décrit dans le document EP 1840582.

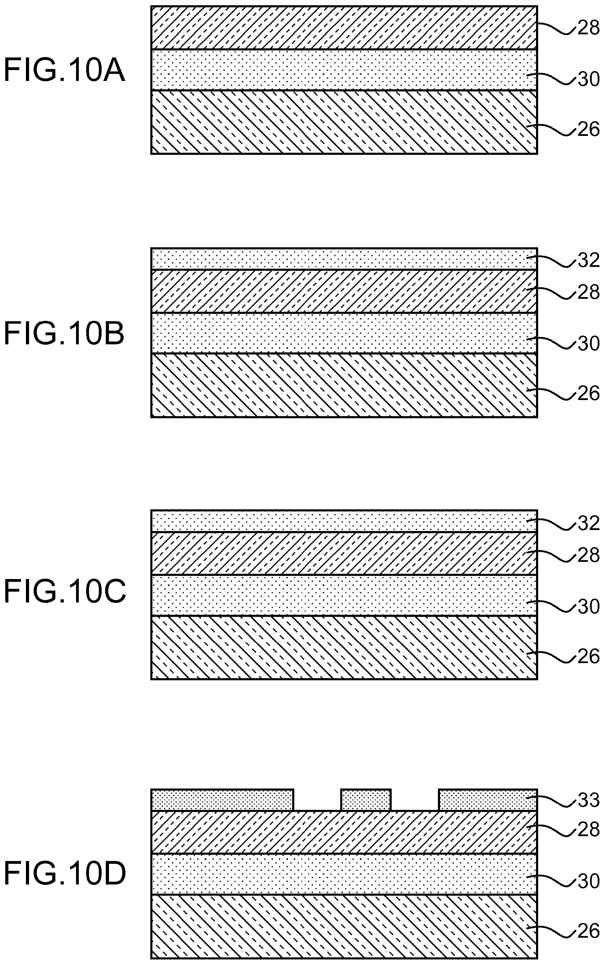

Sur les figures 10A à 10H, on peut voir des représentations schématiques de différentes étapes du procédé de réalisation.

Dans l'exemple décrit, on utilise une plaque de SOI (Silicon On Insulator) en terminologie anglaise ou silicium sur isolant, représenté sur la figure 10A. Le substrat SOI comporte une couche de silicium 26, une couche de silicium monocristallin 28, les couches 26, 28 étant séparées par une couche de $SiO_2$ 30. La couche de silicium 28 monocristallin forme la face avant.

Lors d'une première étape une couche de d'oxyde $SiO_2$ 32 est déposée sur la couche 28. L'élément ainsi formé est représenté sur la figure 10B.

Lors d'une étape suivante, on effectue un dopage P++ par exemple au bore, de la couche de silicium 28 située entre la couche d'oxyde 30 et la couche d'oxyde 32.

Le dopage au travers de la couche d'oxyde permet une répartition plus homogène des dopants dans la couche 28. Le dopage obtenu est de l'ordre de $1.10^{19}$ at./cm$^3$). Ce dopage a pour effet de maximiser le coefficient de température de résistance du silicium.

L'élément ainsi formé est représenté sur la figure 10C. Le dopage est symbolisé par des points.

Lors d'une étape suivante, on retire la couche d'oxyde 32 et on dépose une couche de résine 33, dans laquelle définit les contours des motifs dans la résine 32 par lithographie, par exemple par lithographie en UV profond (DUV pour Deep-UV en en terminologie anglaise) ou par une lithographie hybride DUV et à faisceau d'électrons (e-beam en terminologie anglaise). Ces procédé de lithographie sont bien connus de l'homme du métier et ne seront pas décrits en détail. La lithographie e-beam permet de s'affranchir des effets liés à la diffraction de la lumière lors de la gravure de dispositifs nanométriques.

L'élément ainsi formé est représenté sur la figure 10D.

Lors d'une étape suivante, la couche de silicium est gravée, par exemple par gravure ionique réactive ou RIE (Reactive Ion Etching en anglais) anisotrope.

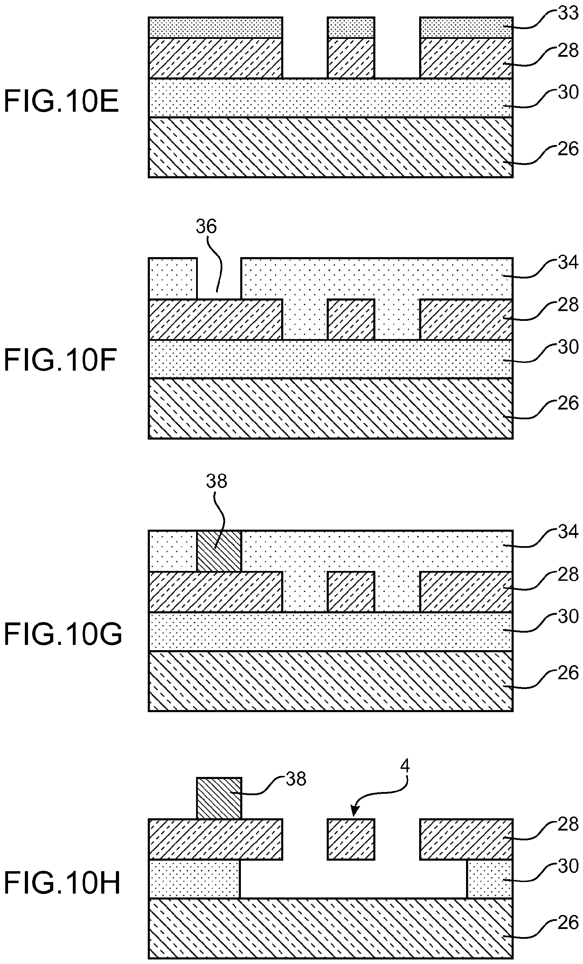

L'élément ainsi formé est représenté sur la figure 10E.

Lors d'une étape suivante, on effectue un dépôt chimique de $SiO_2$ 34 sur la couche de silicium gravée 28 qui est ensuite gravé, par exemple par gravure plasma, pour délimiter les emplacements 36 des contacts électriques. L'élément ainsi obtenu est représenté sur la figure 10F.

De façon optionnelle, il est possible de réaliser deux étapes supplémentaires de lithographie (ebeam par exemple) et de gravure ionique réactive ou RIE (Reactive Ionic Etching) pour nanostructurer le silicium sur les zones d'encastrements des suspensions et/ou des jauges, dans l'exemple de réalisation de la figure 12.

On réalise ensuite les contacts électriques 38 en déposant par exemple de l'aluminium, par exemple par pulvérisation. L'élément ainsi obtenu est représenté sur la figure 10G.

Lors d'une étape suivante, l'élément vibrant est libéré, par exemple en gravant la couche 30, par exemple avec de l'acide fluorhydrique vapeur.

La structure libérée est visible sur la figure 10H.

On obtient alors une structure formée d'un seul bloc de silicium monocristallin suspendu.

Le capteur de flux thermique selon l'invention permet de réaliser un capteur de concentration de gaz.

Comme indiqué ci-dessus ce capteur est particulièrement adapté pour être associé à une microcolonne de chromatographie en phase gazeuse. Un ou plusieurs capteurs sont disposés dans un canal connectée en série en sortie de la microcolonne et permet de détecter les pics d'analytes. En effet, l'élément vibrant isolé thermiquement du substrat peut présenter une constante de temps thermique $\tau$ th plus petite que la largeur des pics de chromatographie, de préférence d'au moins un facteur 10.

**Revendications**

**1.** Capteur de flux thermique comportant

- un support (2),
- au moins un élément (4, 404, 504, 504') destiné à être mis en vibration par rapport au support (2), dit élément vibrant,
- des moyens de suspension (14, 114) et d'isolation thermique dudit élément vibrant (4, 404, 504, 504') par rapport au support (2),
- des moyens de chauffage (10, 410, 510) de l'élément vibrant (4, 404, 504, 504'),
- des moyens d'excitation (8) de l'élément vibrant (4, 404, 504, 504'),
- des moyens de détection (12, 412) de la variation de la fréquence de résonance de l'élément vibrant (4, 404, 504, 504') induite par la variation de température de l'élément vibrant due aux échanges thermiques avec un gaz environnant,

**caractérisé en ce que** lesdits moyens d'excitation sont électrostatiques utilisant une force électrostatique mettant en vibration l'élément vibrant à sa fréquence de résonance, et de sorte à déplacer l'élément vibrant en entier selon une direction dans le plan ou une direction hors-plan.

2. Capteur de flux thermique selon la revendication 1, dans lequel les moyens de suspension (14, 114) et d'isolation thermique comportent au moins une poutre dimensionnée de sorte que les pertes thermiques à travers les moyens de suspension de l'élément vibrant (4, 404, 504, 504') vers le support (2) soient réduites.

3. Capteur de flux thermique selon la revendication 1 ou 2, dans lequel les moyens de suspension et d'isolation thermique (14, 114) comportent au moins deux poutres alignées de part et d'autre de l'élément vibrant (4, 404, 504, 504') ou inclinés l'une par rapport à l'autre et présentant une liaison commune à l'élément vibrant (4, 404, 504, 504').

4. Capteur de flux thermique selon la revendication 2 ou 3, dans lequel la ou les poutres (14, 114) présentent une section comprise entre 10x10 nm$^2$ et 250x250 nm$^2$ et de préférence égale à 50x50 nm$^2$.

5. Capteur de flux thermique selon l'une des revendications 2 à 4, dans lequel la ou les poutres (14, 114) présentent une forme non linéaire, avantageusement en serpentin ou avec au moins un rectangle évidé entre deux tronçons.

6. Capteur selon l'une des revendications 2 à 5, dans lequel la ou les poutres (14, 114) sont en matériau isolant thermique.

7. Capteur selon l'une des revendications 1 à 6, dans lequel les moyens de suspension et d'isolation thermique comportent une zone d'ancrage au support (2), formée par un matériau nanostructuré.

8. Capteur selon l'une des revendications 1 à 7, dans lequel les moyens de chauffage (10, 410, 510) sont des moyens de chauffage par effet Joule.

9. Capteur selon la revendication 8, dans lequel les moyens de chauffage (10, 510) sont en contact direct avec l'élément vibrant (4, 404).

10. Capteur selon la revendication 9, dans lequel les moyens de chauffage (10, 410) sont formés par au moins un élément conducteur électrique relié à une source de polarisation et à l'élément vibrant (4, 404).

11. Capteur selon l'une des revendications 1 à 10, dans lequel les moyens de détection (12) sont formés par au moins une jauge piézorésistive (20.1, 20.2) reliée mécaniquement à l'élément vibrant et/ou les moyens d'excitation (8) sont des moyens d'excitation électrostatique.

12. Capteur selon la revendication 11 en combinaison avec la revendication 10, dans lequel au moins une jauge piézorésistive (20.1, 20.2) forme les moyens de chauffage (10) par effet Joule.

13. Capteur selon l'une des revendications 1 à 7, dans lequel les moyens de chauffage (510) sont situés à distance de l'élément vibrant (504, 504'), le chauffage étant obtenu par conduction à travers l'environnement gazeux entre les moyens de chauffage (510) et l'élément vibrant (504, 504').

14. Capteur selon la revendication 13, dans lequel les moyens de chauffage (510) sont formés par un fil suspendu (522).

15. Capteur selon la revendication 13 ou 14, comportant deux éléments vibrants (504, 504') disposés de part et d'autre des moyens de chauffage (510) de sorte à assurer une mesure différentielle.

16. capteur selon l'une des revendications 1 à 15, dans lequel l'élément vibrant (404) est un élément vibrant selon des modes de déformation en volume.

17. capteur selon l'une des revendications 1 à 16, dans lequel l'élément vibrant (404) est un élément vibrant en rotation.

18. Capteur selon l'une des revendications 1 à 17, dans lequel les moyens de détection (12) comportent deux jauges piézorésistives assurant une mesure différentielle.

19. Capteur selon l'une des revendications 1 à 17, dans lequel les moyens de détection (412) sont des moyens de détection capacitifs.

20. Système de mesure de flux thermique comportant une pluralité de capteurs selon l'une des revendications 1 à 19.

21. Système de détermination de la concentration d'un environnement gazeux comportant au moins un capteur de flux thermique selon l'une des revendications 1 à 19 ou un système selon la revendication 20, une électronique de traitement des valeurs de tension électrique délivrées par le capteur de flux thermique, le au moins un capteur étant avantageusement placé dans une boucle électronique de mise en oscillation et de mesure de la fréquence ou de la phase.

22. Dispositif d'analyse d'un gaz ou mélange de gaz comportant une colonne de chromatographie en phase gazeuse et au moins un système de détermination de la concentration selon la revendication 21, ledit système de détermination étant disposé dans un canal connecté à la sortie de la colonne de chromatographie en phase gazeuse.

23. Procédé de mesure du flux thermique au moyen d'un capteur de flux thermique selon l'une des revendications 1 à 19, comportant :

- une étape d'une mesure unique par le capteur de flux thermique, de la variation de fréquence de l'élément vibrant induite par la variation de température de l'élément vibrant dues aux échanges thermiques avec un gaz environnant.

**Patentansprüche**

1. Wärmeflusssensor, enthaltend

- einen Träger (2),
- zumindest ein Element (4, 404, 504, 504'), Schwingelement genannt, das dazu bestimmt ist, in Bezug auf den Träger (2) in Schwingung versetzt zu werden,
- Mittel (14, 114) zum Aufhängen und Wärmedämmen des Schwingelements (4, 404, 504, 504') relativ zum Träger (2),
- Mittel (10, 410, 510) zum Erwärmen des Schwingelements (4, 404, 504, 504'),
- Mittel (8) zum Erregen des Schwingelements (4, 404, 504, 504'),
- Mittel (12, 412) zum Erfassen der Änderung der Resonanzfrequenz des Schwingelements (4, 404, 504, 504'), die durch die Änderung der Temperatur des Schwingelements aufgrund des Wärmeaustauschs mit einem umgebenden Gas verursacht wird,

**dadurch gekennzeichnet, dass** die Erregermittel elektrostatisch sind und eine elektrostatische Kraft verwenden, die das Schwingelement mit seiner Resonanzfrequenz in Schwingung versetzt, so dass das gesamte Schwingelement in einer Richtung in die Ebene oder in einer Richtung aus der Ebene bewegt wird.

2. Wärmeflusssensor nach Anspruch 1, wobei die Aufhängungs- und Wärmedämmmittel (14, 114) zumindest einen Balken enthalten, der so dimensioniert ist, dass die Wärmeverluste durch die Mittel zum Aufhängen des Schwingelements (4, 404, 504, 504') an den Träger (2) reduziert werden.

3. Wärmeflusssensor nach Anspruch 1 oder 2, wobei die Aufhängungs- und Wärmedämmmittel (14, 114) zumindest zwei zu beiden Seiten des Schwingelements (4, 404, 504, 504') ausgerichtete oder zueinander geneigte Balken enthalten, die eine gemeinsame Verbindung mit dem Schwingelement (4, 404, 504, 504') aufweisen.

4. Wärmeflusssensor nach Anspruch 2 oder 3, wobei der oder die Balken (14, 114) einen Querschnitt zwischen 10 x 10 nm$^2$ und 250 x 250 nm$^2$ und vorzugsweise gleich 50 x 50 nm$^2$ aufweisen.

5. Wärmeflusssensor nach einem der Ansprüche 2 bis 4, wobei der oder die Balken (14, 114) eine nichtlineare Form aufweisen, vorteilhafterweise serpentinenförmig oder mit zumindest einem ausgehöhlten Rechteck zwischen zwei Abschnitten.

6. Sensor nach einem der Ansprüche 2 bis 5, wobei der oder die Balken (14, 114) aus einem wärmedämmenden Material hergestellt sind.

7. Sensor nach einem der Ansprüche 1 bis 6, wobei die Aufhängungs- und Wärmedämmmittel einen Verankerungsbereich zum Verankern mit dem Träger (2) enthalten, der aus einem nanostrukturierten Material gebildet ist.

8. Sensor nach einem der Ansprüche 1 bis 7, wobei die Heizmittel (10, 410, 510) Joule-Effekt-Heizmittel sind.

9. Sensor nach Anspruch 8, wobei die Heizmittel (10, 510) in direktem Kontakt mit dem Schwingelement (4, 404) stehen.

10. Sensor nach Anspruch 9, wobei die Heizmittel (10, 410) aus zumindest einem elektrisch leitenden Element gebildet sind, das mit einer Vorspannungsquelle und mit dem Schwingelement (4, 404) verbunden ist.

11. Sensor nach einem der Ansprüche 1 bis 10, wobei die Erfassungsmittel (12) aus zumindest einem piezoresistiven Messfühler (20.1, 20.2) gebildet sind, der mechanisch mit dem Schwingelement verbunden ist, und/oder wobei die Erregermittel (8) elektrostatische Erregermittel sind.

12. Sensor nach Anspruch 11 in Kombination mit Anspruch 10, wobei zumindest ein piezoresistiver Messfühler (20.1, 20.2) die Joule-Effekt-Heizmittel (10) bildet.

13. Sensor nach einem der Ansprüche 1 bis 7, wobei die Heizmittel (510) von dem Schwingelement (504, 504') beabstandet liegen, wobei die Erwärmung durch Leitung durch die gasförmige Umgebung zwischen den Heizmitteln (510) und dem Schwingelement (504, 504') erreicht wird.

14. Sensor nach Anspruch 13, wobei die Heizmittel (510) aus einem aufgehängten Draht (522) gebildet sind.

15. Sensor nach Anspruch 13 oder 14, enthaltend zwei Schwingelemente (504, 504'), die auf beiden Seiten der Heizmittel (510) angeordnet sind, um eine Differenzmessung sicherzustellen.

16. Sensor nach einem der Ansprüche 1 bis 15, bei dem das Schwingelement (404) ein Element ist, das gemäß Volumenverformungsmodi schwingt.

17. Sensor nach einem der Ansprüche 1 bis 16, wobei das Schwingelement (404) ein rotierendes Schwingelement ist.

18. Sensor nach einem der Ansprüche 1 bis 17, wobei die Erfassungsmittel (12) zwei piezoresistive Messfühler enthalten, die eine Differenzmessung sicherstellen.

19. Sensor nach einem der Ansprüche 1 bis 17, wobei die Erfassungsmittel (412) kapazitive Erfassungsmittel sind.

20. Wärmeflussmesssystem mit einer Vielzahl von Sensoren nach einem der Ansprüche 1 bis 19.

21. System zur Bestimmung der Konzentration einer gasförmigen Umgebung, enthaltend zumindest einen Wärmeflusssensor nach einem der Ansprüche 1 bis 19 oder ein System nach Anspruch 20, eine Elektronik zur Verarbeitung der von dem Wärmeflusssensor gelieferten elektrischen Spannungswerte, wobei der zumindest eine Sensor vorteilhaft in einer elektronischen Schwingungs- und Frequenzmess- oder Phasenmessschleife angeordnet ist.

22. Vorrichtung zur Analyse von einem Gas oder Gasgemisch mit einer Gaschromatographiesäule und zumindest einem Konzentrationsbestimmungssystem nach Anspruch 21, wobei das Bestimmungssystem in einem Kanal angeordnet ist, der mit dem Auslass der Gaschromatographiesäule verbunden ist.

23. Verfahren zum Messen des Wärmeflusses mittels eines Wärmeflusssensors nach einem der Ansprüche 1 bis 19, umfassend:

- einen Schritt einer einzelnen Messung der Frequenzänderung des Schwingelements, die durch die Temperaturänderung des Schwingelements aufgrund der Wärmeaustausche mit einem umgebenden Gas verursacht wird, mittels des Wärmeflusssensors.

## EP 2 711 696 B1

**Claims**

1. A thermal flow sensor comprising:

   - a support (2),
   - at least one element (4, 404, 504, 504') intended to be vibrated relative to the support (2), called vibrating element,
   - suspension and thermal insulation means (14, 114) for insulating said vibrating element (4, 404, 504, 504') relative to the support (2),
   - means (10, 410, 510) for heating the vibrating element (4, 404, 504, 504'),
   - means (8) for exciting the vibrating element (4, 404, 504, 504') so as to vibrate it at its resonance frequency,
   - means (12, 412) for detecting the resonance frequency variation of the vibrating element (4, 404, 504, 504') caused by the temperature variation of the vibrating element due to the heat exchanges with a surrounding gas,

   **Characterized in that** sais means for exciting are electrostatic using an electrostatic force putting the vibrating element into vibration at its resonance frequency, and in such manner that the vibrating element is moved in its entirety along a direction in the plane or in an out-of-plane direction.

2. The thermal flow sensor according to claim 1, wherein the suspension and thermal insulation means (14, 114) comprise at least one beam dimensioned (section, length, shape) such that the heat losses through the suspension means from the vibrating element (4, 404, 504, 504') toward the support (2) are reduced.

3. The thermal flow sensor according to claim 1 or 2, wherein the suspension and thermal insulation means (14, 114) comprise at least two beams aligned on either side of the vibrating element (4, 404, 504, 504') or inclined relative to one another and having a common link to the vibrating element (4, 404, 504, 504').

4. The thermal flow sensor according to claim 2 or 3, wherein the beam (14, 114) has a section comprised between $10 \times 10$ nm$^2$ and $250 \times 250$ nm$^2$, and preferably equal to $50 \times 50$ nm$^2$.

5. The thermal flow sensor according to one of claims 2 to 4, wherein the beam (14, 114) has a nonlinear shape, advantageously the beam has a serpentine shape or a shape with at least one rectangle that is hollow between two segments.

6. The sensor according to claim 2, wherein the beam(s) is (are) made from a thermally insulating material.

7. The sensor according to one of the claims 1 to 6, wherein the suspension and thermal insulation means comprise a zone for anchoring to the support (2), formed by a nanostructured material.

8. The sensor according to claim 1 or 2, wherein the heating means (10, 410, 510) are heating means by Joule effect.

9. The sensor according to claim 9, wherein the heating means (10, 510) are in direct contact with the vibrating element (4, 404).

10. The sensor according to claim 9, wherein the heating means (10, 410) are formed by at least one electrically conducting element connected to a polarization source and the vibrating element (4, 404).

11. The sensor according to one of the claim 1 to 10, wherein the detection means (12) are formed by at least one piezoresistive gauge (20.1, 20.2) mechanically connected to the vibrating element and/or the excitation means (8) are electrostatic excitation means.

12. The sensor according to claim 11 in combination with claim 10, wherein at least one piezoresistive gauge (20.1, 20.2) forms the Joule effect heating means (10).

13. The sensor according to claim 1 or 2, wherein the heating means (510) are situated separated from the vibrating element (504, 504'), the heating being obtained by conduction through the gaseous environment between the heating means (510) and the vibrating element (504, 504').

14. The sensor according to claim 13, wherein the heating means (510) are formed by a suspended wire (522).

17

**15.** The sensor according to claim 13 or 14, comprising two vibrating elements (504, 504') positioned on either side of the heating means (510) so as to perform a differential measurement.

**16.** The sensor according to one of the claims 1 to 15, wherein the vibrating element (404) is a vibrating element according to volume deformation modes.

**17.** The sensor according to one of the claims 1 to 16, wherein the vibrating element (404) is a rotational vibrating element.

**18.** The sensor according to one of the claims 1 to 17, wherein the detection means (12) comprise two piezoresistive gages providing a differential measurement.

**19.** The sensor according to one of the claims 1 to 17, wherein the detection means (412) are capacitive detection means.

**20.** A thermal flow measuring system comprising a plurality of sensors according to one of claims 1 to 19.

**21.** A system for determining the concentration of a gaseous environment comprising at least one thermal flow sensor according to one of claims 1 to 19 or a system according to claim 20, electronics for processing the electrical voltage values delivered by the thermal flow sensor, at least one sensor being advantageously placed in an electronic oscillating and frequency- or phase-measuring loop.

**22.** A device for analyzing a gas or mixture of gases comprising a gas chromatography column and at least one system for determining the concentration according to claim 21, said determination system being positioned in a channel connected to the outlet of the gas chromatography column.

**23.** A method for measuring the thermal flow using a thermal flow sensor according to one of claims 1 to 19, comprising:

- a step for a single measurement by the thermal flow sensor of the frequency variation of the vibrating element caused by the temperature variation of the vibrating element due to the heat exchanges with a surrounding gas.

FIG.1

EP 2 711 696 B1

FIG.2A

FIG.2B

FIG.3

FIG.4A

FIG.4B

FIG.4C

FIG.5

FIG.6A

FIG.6B

FIG.6C

FIG.6D

FIG.7

FIG.8A

FIG.8B

FIG.8C

FIG.9

EP 2 711 696 B1

FIG.10A

FIG.10B

FIG.10C

FIG.10D

FIG.10E

33
28
30
26

FIG.10F

36
34
28
30
26

FIG.10G

38
34
28
30
26

FIG.10H

38
4
28
30
26

FIG.11

FIG.12

FIG.13A

FIG.13B

FIG.14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1840582 A

**Littérature non-brevet citée dans la description**

- **FANGET, S. HENTZ ; P. PUGET ; J. ARCAMONE ; M. MATHERON ; E. COLINET ; P. ANDREUCCI ; L. DURAFFOURG ; E. MYERS ; M.L. ROUKES.** Gas sensors based on gravimétrie detection - A review. *Sensors and Actuators, B: Chemical,* 2011, vol. 160 (1), 804-821

- **D. MERCIER et al.** Characterization of a SAW-Pirani vacuum sensor for two différent operating modes. *Sensors and actuators, A: Physical,* 2012, vol. 188, 41-47